(19) Europäisches Patentamt
European Patent Office
Office européen des brevets

(11) Numéro de publication : 0 283 400 B1

# (12) FASCICULE DE BREVET EUROPEEN

(45) Date de publication du fascicule du brevet : 20.03.91 Bulletin 91/12

(51) Int. Cl.$^5$ : C07C 6/04, B01J 31/02, B01J 31/34

(21) Numéro de dépôt : 88400622.2

(22) Date de dépôt : 16.03.88

## (54) Systèmes catalytiques perfectionnés à durée de vie et de conservation prolongées pour la métathèse d'oléfines.

(30) Priorité : 20.03.87 FR 8703879

(43) Date de publication de la demande : 21.09.88 Bulletin 88/38

(45) Mention de la délivrance du brevet : 20.03.91 Bulletin 91/12

(84) Etats contractants désignés : BE CH DE GB IT LI NL

(56) Documents cités :
EP-A- 0 191 675
FR-A- 2 547 513
FR-A- 2 565 505
GB-A- 1 232 831
US-A- 4 078 013
US-A- 4 201 730
US-A- 4 262 156

(73) Titulaire : SOCIETE NATIONALE ELF AQUITAINE
Tour Elf, 2, Place de la Coupole, La Défense 6
F-92400 Courbevoie (FR)

(72) Inventeur : Laval, Jean-Paul
16 rue Aquitaine
F-31120 Roquette (FR)
Inventeur : Leconte, Michel
26 rue Jean-Claude Vivant
F-69100 Villeurbanne (FR)
Inventeur : Ollivier, Jean
Croix de Buzy
F-64260 Arudy (FR)
Inventeur : Perez, Emile
17 Allée d'Aspin
F-31770 Colomiers (FR)
Inventeur : Quignard, Françoise
15 rue du Charriot d'Or
F-69004 Villeurbanne (FR)
Inventeur : Rico, Isabelle
12 rue S. Garcia
F-31520 Ramonville Saint Agne (FR)

(74) Mandataire : Kohn, Armand
5 Avenue Foch
F-92380 Garches (FR)

EP 0 283 400 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

**Description**

La présente invention concerne des nouveaux systèmes catalytiques à longue durée de vie, un procédé de stabilisation et/ou de conservation de leur activité catalytique au cours du temps après leur préparation, ainsi que l'application de ces systèmes catalytiques à la métathèse de différentes oléfines.

La métathèse, c'est-à-dire dismutation, d'oléfines a fait l'objet, ces temps derniers, de nombreuses études, du fait de l'intérêt pratique qu'elle présente. Elle comprend l'échange de groupements aboutissant à la double liaison, entre deux molécules d'oléfines, d'où la possibilité de produire des composés difficiles à synthétiser par des voies connues, et le grand intérêt industriel qui en découle de ce mode de réaction. L'opération s'effectuant par catalyse hétérogène ou homogène, les systèmes catalytiques, généralement employés, sont à base de métaux de transition tels que W, Mo ou Re. La réaction peut s'écrire schématiquement :

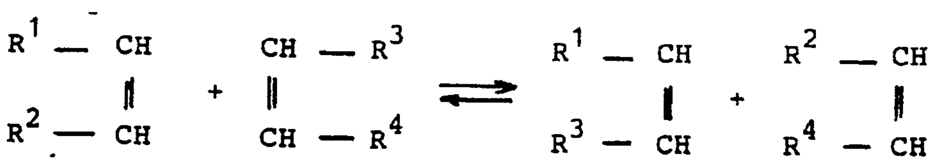 (1)

$R^1$ à $R^4$ désignant des chaînes ou groupes carbonés, dont certains peuvent être semblables, pouvant porter des fonctions telles que, par exemple, carboxyles, hydroxyles, amines, amides, nitriles, silyles, halogènes, éthers, esters ou autres.

Des systèmes catalytiques homogènes, souvent utilisés et particulièrement adaptés à la métathèse d'oléfines porteuses ou non de groupes fonctionnels, sont constitués, par exemple, par les systèmes associant des composés du tungstène, qui possèdent des atomes d'halogènes et/ou des ligandes aryloxy, à des composés organométalliques, tels qu'organo-stanniques, -plombiques, -aluminiques, -lithiens ou -magnésiens. De tels systèmes sont décrits, notamment dans l'article de J. Otton, Y. Colleuille et J. Varagnat, paru dans Journal of Molecular Catalysis, 8, 1980, p. 313-324, ainsi que dans les demandes de brevet français N° 2 547 513 et 2 565 505.

La demande N° 2 547 513 vise les catalyseurs du type :

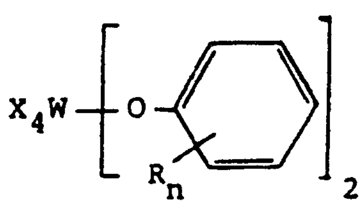

où X est un halogène, R un groupe hydrocarboné, groupe ou atome électronégatif, et n un nombre entier de 1 à 5. De tels catalyseurs associés à des composés de Al ou Sn ont permis de dismuter diverses oléfines. Selon la demande 2 565 505 l'adjonction de composés de Pb est préconisée au lieu de composés de Sn.

Bien que ces systèmes catalytiques soient doués d'une activité satisfaisante, ils présentent cependant l'inconvénient majeur de ne conserver cette activité que pendant un laps de temps relativement court, après la mise en présence de leurs constituants.

En conséquence, leur emploi nécessite leur préparation "in situ", au moment du démarrage de la réaction catalytique. Leur mise en oeuvre notamment dans des procédés à l'échelle industrielle présente, de ce fait, un inconvénient.

La présente invention a pour but de remédier à cet inconvénient. Elle apporte, à cet égard, un progrès important.

C'est ainsi que la demanderesse a trouvé et c'est précisément l'un des objets de l'invention, qu'il était possible de conserver des systèmes catalytiques de métathèse à base de composés de tungstène, plus d'une dizaine de jours avant leur mise en oeuvre, tout en maintenant constante leur activité, cela grâce à l'incorporation d'un composé supplémentaire, que l'art antérieur ne pouvait en rien laisser prévoir.

La présente invention se caractérise principalement par le fait que ce composé supplémentaire est un hydrocarbure saturé ou non, comportant une chaîne hydrocarbonée partiellement ou totalement fluorée.

Le système catalytique perfectionné, suivant l'invention, pour la métathèse d'oléfines, qui comprend au départ (a) un composé halogéné du tungstène, (b) un composé organométallique, alkylique ou arylique, d'un métal autre que w, et (c) un composé organique halogéné, est caractérisé en ce que le composé (c) est un

hydrocarbure aliphatique fluoré ou perfluoré.

Le composant (c) est plus particulièrement un composé organique fluoré, saturé ou insaturé de formule générale :

$$R_F — R'' — R'_F$$

dans laquelle R" est une chaîne hydrocarbonée saturée ou insatureé, possédant de 0 à 7 atomes de carbone, $R_F$ et $R'_F$ identiques ou différents entre eux sont des chaînes carbonées saturées ou insaturées partiellement ou totalement fluorées, de préférence carbonées, saturées, totalement fluorées, $R_F$ pouvant posséder de 0 à 20 atomes de carbone, de préférence 0 à 12 atomes, et $R'_F$ de 2 à 20 atomes de carbone, de préférence 2 à 12 atomes.

R" représente, de préférence, une chaîne hydrocarbonée saturée ou insaturée, choisie parmi : $CH_3\text{-}(CH_2)_{\overline{n}}$ ou $-(CH_2)_{n'}$, -n étant un nombre entier de 0 à 6 et n' un nombre entier de 0 à 5 ; $CH_2\text{=}CH\text{-}(CH_2)_{\overline{m}}$ m étant un nombre entier de 0 à 3 ; ou –(CH=CH)–.

Parmi les composants (c), qui peuvent être avantageusement utilisés, on peut citer la liste suivante :

$C_8F_{17}C_2H_5$ ; $C_4F_9\text{–}CH\text{=}CH_2$ ; $C_6F_{13}\text{–}CH\text{=}CH_2$ ;
$C_8F_{17}CH\text{=}CH_2$ ; $C_6F_{13}CH\text{=}CHC_6F_{13}$.

Le composant (a) a pour formule générale :

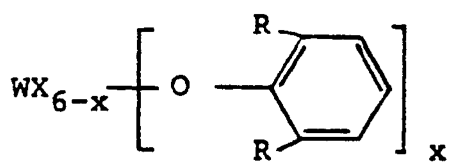

où x est égal à 0 ou 2 ; X est un halogène, de préférence du chlore ou du fluor ; R est un groupe hydrocarboné ou un groupe ou atome électronégatif, de préférence un alkyle en $C_1$ à $C_6$, phényle ou un atome de Cl, Br ou F.

Le composant (b), organométallique associé au composant (a), peut être l'un de ceux où l'atome de métal est, entre autres, du Li, Mg, Ti, Al, Sn ou Pb, plus particulièrement des organo-aluminiques, -stanniques ou -plombiques et, de préférence, des composés de formule $M(R')_4$ où M est un atome de Sn ou de Pb et R' un groupement alkyle en $C_1$ à $C_{12}$ ou aryle en $C_6$ à $C_{18}$ et, plus spécialement, un alkyle en $C_1$ à $C_5$.

Selon l'invention, le rapport molaire composant (c)/composant (a), dans le système catalytique initial, est compris entre 0,5/1 et 20/1 et, de préférence de 0,5/1 à 1/1, tandis que le rapport molaire composant (b)/composant (a) peut être toute valeur existante dans les catalyseurs connus et, plus particulièrement, de 1/1 à 4/1, de préférence d'environ 2.

Il est approprié que les systèmes catalytiques de la présente invention soient en solution dans un solvant organique, ce qui est propice à la catalyse en phase homogène. Parmi les très nombreux solvants qui peuvent convenir, on peut mentionner notamment le benzène, toluène, les fréons, les hydrocarbures saturés en $C_6$ à $C_{22}$ et, de préférence, du chlorobenzène.

Le procédé de stabilisation et/ou de conservation de l'activité des nouveaux systèmes catalytiques de l'invention comprend : la mise en solution du composé du tungstène, c'est-à-dire le composant (a), dans un solvant organique approprié ; la mise en contact du composé organo-métallique (b) avec le composant (a), l'agitation et le chauffage à une température entre 0° et 100°C du mélange réactionnel qui en résulte, pendant 0,5 à 60 minutes, puis introduction d'une quantité efficace du produit organique fluoré, ou composant (c), et arrêt du chauffage et de l'agitation.

Les proportions utiles du composant (c) introduit correspondent à un rapport molaire composant (c)/composant (a) de 0,5/1 à 20/1. Des proportions molaires supérieures à 20/1, par exemple 50/1 sont employables quoique rarement justifiables économiquement.

Selon une forme préférée de réalisation du procédé de stabilisation de l'invention, le temps de chauffage est de 0,5 à 10 minutes lorsque le composant (b) est un organo-plombique, et de 0,5 à 30 minutes dans le cas d'un organo-stannique.

Le solvant organique mis en oeuvre dans le procédé est, plus particulièrement, du benzène, toluène, des fréons, des hydrocarbures saturés en $C_6$ à $C_{22}$ et, de préférence du chlorobenzène.

Les rapports molaires composant (b)/composant (a) de ces constituants mis au contact l'un de l'autre sont avantageusement compris entre 1/1 et 4/1 et, de préférence, d'environ 2.

La présente invention a également pour objet l'application des systèmes catalytiques, ainsi stabilisés, à différents types de réactions dans le domaine de la métathèse. Ces réactions peuvent concerner aussi bien des oléfines acycliques, fonctionnelles ou non, plus particulièrement des esters oléfiniques, aussi bien que des oléfines cycliques. Dans ces réactions, les rapports molaires oléfines/composant(a) du système catalytique peuvent être compris entre 10 et 10000 et, de préférence entre 50 et 2000. Ces réactions peuvent être conduites à des températures comprises entre 0°C et 150°C et, plus particulièrement, entre 25°C et 85°C.

Bien entendu, la solution catalytique, selon l'invention, peut être utilisée immédiatement après sa préparation. Cependant, le mérite de l'invention qui constitue une très nette supériorité par rapport à l'art antérieur, est que cette solution peut être conservée, en l'état, plus d'une dizaine de jours. Elle peut alors être mise en oeuvre dans des réactions de métathèse d'oléfines sans que l'on ne note une quelconque diminution d'activité catalytique par rapport à l'activité observée immédiatement après la stabilisation du système catalytique selon l'invention. Bien au contraire, il apparaît de façon surprenante que la grande stabilité du système catalytique préparé, selon l'invention, permet de convertir en un temps donné, et pour une quantité donnée de catalyseur, un plus grand nombre de molécules d'oléfines que ne le font les systèmes catalytiques de l'art antérieur. De plus, il est possible, après avoir fait réagir une charge d'oléfine, de ré-introduire dans le milieu réactionnel une nouvelle charge et de la convertir, sans que l'on n'observe une diminution de l'activité du système catalytique, et cela sans modifier en quoi que ce soit le milieu réactionnel. Cette nouvelle charge étant elle-même convertie, on peut procéder à un nouveau cycle de transformation. Ce type d'opération peut être répété plusieurs fois, avant que l'on n'observe une diminution appréciable de l'activité du système catalytique de l'invention.

Les exemples suivants ont pour seul but d'illustrer les diverses possibilités, les domaines d'application et les avantages de la présente invention par rapport à l'art antérieur.

## EXEMPLES 1 à 4

### Système catalytique stabilisé selon l'invention

On met en contact $2.10^{-3}$ mole de tétrabutylétain ($SnBu_4$) avec $10^{-3}$ mole de

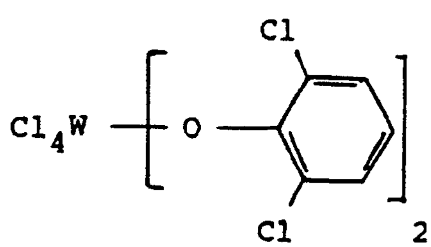

dans 10 ml de chlorobenzène à une température de 85°C. Après 20 minutes d'agitation, on y ajoute $10^{-3}$ mole de composé fluoré de formule $C_8F_{17}$-CH=$CH_2$. La solution catalytique, ainsi obtenue, est laissée au repos à la température ambiante.

### Application de ce système catalytique à la métathèse de pentène-2-cis

Cette réaction qui donne du butène-2 et de l'héxène-3 peut s'écrire schématiquement :

$$2CH_3\text{-}CH = CH\text{-}C_2H_5 \rightleftarrows CH_3\text{-}CH{=}CH\text{-}CH_3 + C_2H_5\text{-}CH = CH\text{-}C_2H_5$$

Après des temps variables, indiqués dans le Tableau I, on prélève 1 ml de la solution catalytique sus-indiquée, correspondant à $10^{-4}$ mole de composé tungstènique, $2.10^{-4}$ mole de composé stannique et $10^{-4}$ mole de composé organique fluoré, que l'on introduit dans un réacteur opérant en discontinu, préalablement purgé à l'argon, et maintenu à 85°C, avec $50.10^{-4}$ mole de pentène-2-cis en solution dans 5 ml de chlorobenzène. La conversion de pentène-2-cis est suivie par l'analyse de la quantité de butène-2 formé.

EXEMPLES 5 et 6

Comparaison avec un système catalytique de l'art antérieur décrit dans la demande de brevet français N° 2 547 513

Dans un réacteur où l'on opère en discontinu, préalablement purgé à l'argon, on introduit $10^{-4}$ mole du composé tungsténique des exemples 1 à 4. Ensuite, on y verse 5 ml de chlorobenzène servant de solvant. On ajoute à la solution, ainsi formée, $2.10^{-4}$ mole de tétrabutylétain. Le mélange réactionnel est agité et chauffé à 85°C pendant 20 minutes. Dans le cas de l'exemple 5 on y introduit alors $50.10^{-4}$ mole de pentène-2-cis, et l'on suit la réaction de métathèse à 85°C, comme pour les exemples 1 à 4, par l'analyse de la quantité de butène-2 formé. Dans le cas de l'exemple 6, le pentène-2-cis est ajouté 18 heures après la préparation de la solution catalytique.

Les résultats des exemples 1 à 6, rassemblés dans le Tableau I, montrent que les systèmes catalytiques des exemples 1 à 4, objet de l'invention, permettent d'atteindre l'équilibre de la réaction de métathèse du pentène-2-cis même lorsque le temps écoulé entre la stabilisation du système catalytique et l'utilisation de ce dernier est de 10 jours. Par contre, le système catalytique de l'art antérieur (exemples 5 et 6) ne permet d'atteindre l'équilibre de la réaction de métathèse que s'il est utilisé immédiatement après sa préparation.

TABLEAU I

| Exemples | Systèmes Catalytiques | t(h) * | $t_r$ (mn) ** | Rendement en butène *** (mole %) |
|---|---|---|---|---|
| 1 | $WCl_4$ 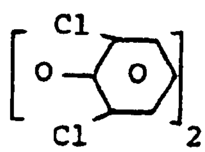 $/SnBu_4/$ $C_8F_{17}-CH=CH_2$ | 18h | 45 mn | 23% |
| 2 | " " " | 93h | 60 mn | 24% |
| 3 | " " " | 160h | 60 mn | 24% |
| 4 | " " " | 240h | 60 mn | 24% |
| 5 | $WCl_4$ 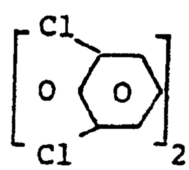 $/SnBu_4$ | 0 | 90 mn | 23% |
| 6 | " " " | 18h | 90 mn | 5% |

*) t : temps écoulé entre la stabilisation du système catalytique et son utilisation dans la métathèse du pentène 2-cis.

**) $t_r$ : durée de réaction du pentène 2-cis en présence du système catalytique.

***) Il est connu que lorsque l'équilibre thermodynamique de la métathèse est atteint, le pentène-2 initial est transformé en 25% de butène-2, 25% d'hexène 3 et 50% de pentène-2.

Ces résultats sont à comparer également avec celui de l'exemple 42.

EXEMPLES 7 à 9

Ces exemples décrivent la métathèse du pentène-2-cis avec des systèmes catalytiques stabilisés comme indiqué dans les exemples 1 à 4 conformes à l'invention, dans lesquels pour chacun des exemples 7 à 9, le composant (c) stabilisant organique fluoré est différent pour un même composant (a) et un même composant (b). Les essais consistent à suivre la métathèse du pentène-2-cis comme il a été indiqué dans les exemples 1 à 6. La stabilisation de la solution catalytique est conduite en tout point selon le procédé décrit pour les exemples 1 à 4, en particulier on utilise la même quantité molaire du composé organique fluoré que la quantité du composé tungsténique. Les différents composés organiques fluorés utilisés sont reportés dans le tableau II, page 12, à la colonne composant (c). Dans les exemples 7 à 9, les différentes solutions catalytiques utilisées l'ont toutes été 18 h après leur préparation, la température de réaction de métathèse étant de 85°C.

Les résultats obtenus sont reportés dans le Tableau II où figurent les mêmes abréviations que celles du Tableau I pour les exemples 1 à 6. Ces résultats montrent qu'un système catalytique stabilisé, selon l'invention, peut l'être avec plusieurs types de composés organiques fluorés. Le nouveau système catalytique conserve la même activité avec des stabilisants (composé (c)) où la longueur de la chaîne carbonée fluorée est différente (exemples 1 et 8), la partie hydrocarbonée étant saturée ou non saturée (exemples 1 et 7). L'utilisation d'oléfines fluorées internes et symétriques comme stabilisants, conduit à une conversion du pentène-2 comparable à la conversion obtenue au moyen d'un système catalytique à oléfine fluorée terminale (exemples 1 et 9).

**TABLEAU II**

| Exemples | Systèmes catalytiques | | | $t_r$ (mn) | Rendement (mole %) |
|---|---|---|---|---|---|
| | Composant (a) | Composant (b) | Composant (c) | | |
| 1 | $WCl_4$ 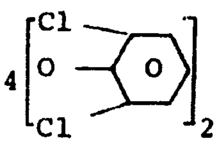 | $SnBu_4$ | $C_8F_{17}-CH=CH_2$ | 45 | 23% |
| 7 | " " | " " | $C_8F_{17}-CH_2-CH_3$ | 90 | 24% |
| 8 | " " | " " | $C_4F_9-CH=CH_2$ | 90 | 23% |
| 9 | " " | " " | $C_8F_{17}-CH=CH-C_8F_{17}$ | 90 | 18% |

EXEMPLES 10 à 13

Des systèmes catalytiques conformes à l'invention, comportant les mêmes composants, sont stabilisés suivant les exemples 1 à 4, mais diffèrent, cependant, l'un de l'autre par la variation du rapport molaire composant (c)/composant (a), alors que le rapport molaire commosant (b)/composant (a) reste égal à environ 2.

On applique ces 4 systèmes catalytiques à la métathèse du pentène-2-cis à 85°C, 18 heures après leur stabilisation. Les résultats consignés dans le tableau III, page 14, (mêmes abréviations que dans le Tableau I), montrent que le produit organique fluoré peut être utilisé avantageusement comme stabilisant dans un large domaine de rapports molaires initiaux composant (c)/composant (a) (compris entre 0,5 et 20), ce qui apporte une grande souplesse à son emploi.

TABLEAU III

| Exemples | Système catalytique composants initiaux(a)+(b)+(c) | Rapports molaires initiaux composants (c)/(a) | $t_r$ (mn) | Rendement butène % molaire |
|---|---|---|---|---|
| 10 | $WCl_4$ 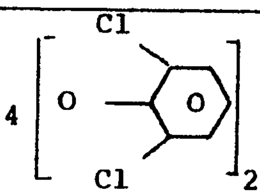 | 0,5 | 20 | 21 |
| 1 | + | 1 | 45 | 23 |
| 11 | Sn $Bu_4$ | 5 | 45 | 24 |
| | + | | | |
| 12 | $C_8F_{17}-CH=CH_2$ | 20 | 45 | 20 |
| 13 | | 50 | 45 | 2 |

EXEMPLES 14 à 18

On applique le système catalytique des exemples 1 à 4 à la métathèse du pentène-2-cis à différentes températures, dans les exemples 14 à 16. A titre de comparaison, on a effectué les exemples 17 et 18 où cette métathèse, opérée dans des conditions opératoires semblables, est faite avec le système catalytique des exemples 5-6 de l'art antérieur. Les résultats de ces essais sont résumés dans le Tableau IV, (mêmes abréviations que dans le Tableau I).

On peut remarquer que le système catalytique de l'invention est aussi actif à 25°C qu'à 85°C et ce, quelle que soit la durée de conservation du système catalytique entre sa stabilisation et son utilisation (exemples 14 à 16 et 3). De plus, on constate une nette amélioration du rendement en butène à la température ambiante (25°C) par rapport au catalyseur de l'art antérieur (exemples 17-18).

TABLEAU IV

| Exemples | Systèmes catalytiques | Température de réaction catalytique | t (h) | tr (mn) | Rendement en butène (mole %) |
|---|---|---|---|---|---|
| 14 | $WCl_4$ 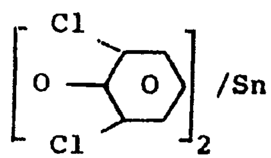 /Sn $Bu_4$/ $C_8F_{17}CH=CH_2$ des exemples 1 à 4. | 25°C | 1h | 60 | 22% |
| 15 | idem | 25°C | 160h | 90 | 24% |
| 16 | idem | 65°C | 160h | 60 | 24% |
| 3 | idem | 85°C | 160h | 60 | 24% |
| 17 | $WCl_4$ 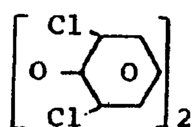 /Sn $Bu_4$ des exemples 5-6 | 25°C | 1h | 60 | 4% |
| 18 | idem | 25°C | 160h | 90 | 0% |

EXEMPLES 19 à 23

On applique le système catalytique stabilisé conformément aux exemples 1 à 4, à la métathèse du pentène-2-cis à 25°C à des temps variables après sa stabilisation, en mettant en oeuvre un rapport molaire pentène-2-cis/composant(a) de 100 ou 1000. Les résultats sont résumés dans le tableau V, (mêmes abréviations que dans le Tableau I). On a également fait figurer dans le Tableau V l'exemple 15 (rapport molaire oléfine/composant (a) égal à 50).

A titre comparatif (exemples 22-23), on a employé un système catalytique suivant la demande de brevet français N° 2 346 047 laquelle propose d'ajouter un composé contenant au moins une fonction éther à un système catalytique à base de complexes du tungstène. La solution catalytique a été préparée comme indiquée dans cette publication, puis employée, à des temps variables après sa préparation, et à différentes températures. Les rapports molaires pentène-2-cis/composant tungsténique sont de 1000.

Dans les exemples 15 et 19 à 23, les composants (a) et (b) du système catalytique sont respectivement :

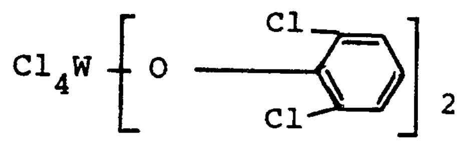

et tétrabutylétain Sn $Bu_4$.

Le rapport molaire composants (a)/(b)/(c) est de 1/2/1. Le composant (c) est identifié dans le Tableau V pour chacun des exemples.

On observe dans le Tableau V de résultats, que même à 25°C, et quel que soit le temps écoulé entre la stabilisation du système catalytique, selon l'invention, et sa mise en oeuvre, l'activité de ce système demeure identique pour les trois rapports molaires oléfine/composant (a) de 50, 100 et 1000 (exemples 15,19 à 21). Alors qu'avec le système catalytique de l'art antérieur, stabilisé à l'éther (exemples 22-23), on remarque que son activité diminue pour des rapports molaires oléfine/composant(a) relativement élevés, notamment 1000. Même en élevant la température de la réaction de métathèse de 25° à 65°C, le rendement en butène reste pratiquement inchangé (exemple 23).

Ces résultats comparatifs montrent que les systèmes catalytiques de l'invention sont considérablement plus actifs que ceux de l'art antérieur représentés par la demande de brevet français 2 346 047.

TABLEAU V

| Exemples | Stabilisants | t(h) | Rapports molaires oléfine/composant(a) | T(°C) | $tr_{(mn)}$ | Rendement en butène mole % |
|---|---|---|---|---|---|---|
| 15 | $C_8F_{17}$ $CH=CH_2$ | 160 | 50 | 25 | 90 | 24% |
| 19 | idem | 20 | 1000 | 25 | 90 | 24% |
| 20 | idem | 160 | 1000 | 25 | 90 | 24% |
| 21 | idem | 240 | 100 | 25 | 90 | 24% |
| 22 | $(C_2H_5)_2O$ | 18 | 1000 | 25 | 90 | 9% |
| 23 | $(C_2H_5)_2O$ | 160 | 1000 | 65 | 90 | 10% |

EXEMPLES 24 à 26

On applique le système catalytique de l'exemple 1 à la métathèse à 25°C du pentène-2-cis utilisant un rapport molaire pentène-2-cis/composé tungsténique(a) égal à 100. Après une heure de réaction, on introduit dans le milieu réactionnel une nouvelle charge d'oléfine identique à la première. Après une nouvelle heure, on analyse le résultat de la réaction globale, c'est-à-dire de la première et de la deuxième charge cumulées, et un nouveau cycle opérationnel est effectué.

A titre de comparaison, on prépare deux solutions catalytiques, selon l'art antérieur (exemples 25 et 26), contenant les mêmes composants (a) et (b) et dans les mêmes proportions molaires que dans l'exemple 24, sauf que dans l'exemple 25 il n'y a aucun composant (c), tandis que dans l'exemple 26 le composant (c) est de l'éther.

Les solutions catalytiques des exemples 25 et 26 sont alors soumises aux mêmes conditions opératoires de métathèse du pentène-2-cis que l'exemple 24.

Les résultats, consignés dans le Tableau VI (mêmes abréviations que dans le Tableau I), montrent que seule la solution catalytique stabilisée au moyen du composé organique fluoré selon l'invention (exemple 24) permet d'atteindre, à chaque charge d'oléfine, l'équilibre thermodynamique de la réaction, sans que l'on observe une diminution de l'activité catalytique.

TABLEAU VI

| Exemples | Stabilisants | t (h) | Rapport molaire pentène-2-cis / composant (a) | tr (mn) | Rendement en butène (mole %) total |
|---|---|---|---|---|---|
| 24 | $C_8F_{17}CH=CH_2$ | 72 | 100<br>+100<br>+100<br>+100<br>+100 | 60<br>60<br>60<br>60<br>60 | 22 %<br>22 %<br>23 %<br>23 %<br>23 % |
| 25 | sans | 0,3 | 100<br>+100<br>+100 | 60<br>60<br>60 | 4 %<br>6 %<br>10 % |
| 26 | $(C_2H_5)_2O$ | 0,3 | 100<br>+100<br>+100 | 60<br>60<br>60 | 22 %<br>16 %<br>12 % |

EXEMPLES 27 à 32

On met en oeuvre différents systèmes catalytiques stabilisés, comme indiqué dans l'exemple 1, et comportant un rapport molaire des composants (a)/(b)/(c) égal à 1/2/1 pour les exemples 28, 30 et 32. La métathèse de pentène-2-cis est effectuée à 85°C avec un rapport molaire pentène-2-cis/composant (a) de 50.

A titre de comparaison, on a utilisé des solutions catalytiques de l'art antérieur (exemples 27, 29 et 31) renfermant les mêmes composants (a) et (b), dans les mêmes rapports molaires que pour les systèmes catalytiques de l'invention (exemples 28, 30 et 32), mais sans composant (c) (organofluoré).

Les résultats de la métathèse ont été résumés dans le Tableau VII, (mêmes abréviations que dans le Tableau I). Ils montrent que la conversion du pentène-2-cis en butène-2 est bien supérieure lorsqu'on exécute la réaction au moyen des solutions catalytiques de l'invention, et ce, quelle que soit la nature des composants (a) et (b).

TABLEAU VII

| Exemples | Composé tungsténique (a) | Composant (b) | Composant (c) | t (h) | $t_r$ (mn) | Rendement en butène (mole %) |
|---|---|---|---|---|---|---|
| 27 | 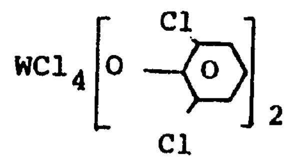 | $Sn(CH_3)_4$ | sans | 18 | 60 | 15 % |
| 28 | idem | idem | $C_8F_{17}CH=CH_2$ | 18 | 60 | 25% |
| 29 | 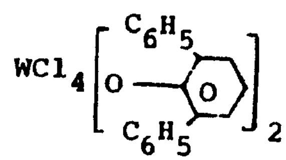 | $Pb(C_4H_9)_4$ | sans | 18 | 30 | 7% |
| 30 | idem | idem | $C_8F_{17}CH=CH_2$ | 18 | 30 | 21% |
| 31 | $WCl_6$ | $Sn(CH_3)_4$ | sans | 68 | 90 | 0% |
| 32 | idem | idem | $C_8F_{17}CH=CH_2$ | 68 | 90 | 20% |

EXEMPLES 33 à 35

On effectue la métathèse d'une oléfine porteuse de groupes fonctionnels (oléate d'éthyle) avec un système catalytique selon l'invention que l'on compare avec un système catalytique de l'art antérieur.

Les essais consistent à maintenir à 85°C de l'oléate d'éthyle, en présence d'un système catalytique selon l'invention (exemple 33) ou préparé selon l'art antérieur (exemples 34 et 35) et à déterminer le % d'octadécène-9 formé suivant la réaction :

$$2CH_3(CH_2)_7CH{=}CH(CH_2)_7COOC_2H_5 \rightleftarrows CH_3(CH_2)_7CH{=}CH(CH_2)_7CH_3 + C_2H_5OOC(CH_2)_7CH{=}CH(CH_2)_7COOC_2H_5$$

qui fournit en même temps de l'hexadécène-8-di-1,16-carboxylate d'éthyle.

Dans les exemples 34 et 35, la métathèse de l'oléate d'éthyle est réalisée avec le système catalytique

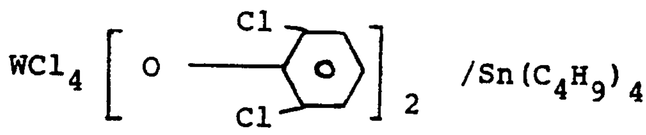

de l'art antérieur selon des conditions analogues à celles utilisées dans le cas de la métathèse du pentène-2-cis et décrites dans les exemples 5 et 6 précédents. Cette solution catalytique est utilisée soit immédiatement (exemple 34), soit 18 heures (exemple 35) après sa préparation.Dans l'exemple 33 la métathèse de l'oléate d'éthyle est réalisée en utilisant le système catalytique

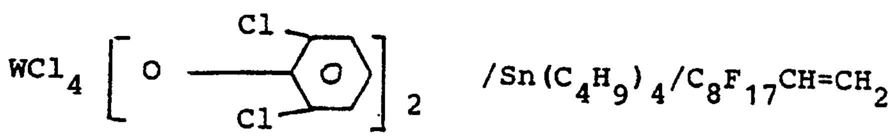

préparé selon l'invention, et dans des conditions analogues à celles utilisées dans le cas de la métathèse du pentène-2-cis et décrites dans les exemples 1 à 4. La solution catalytique a été utilisée 18 heures après sa préparation.

Dans les trois exemples 33 à 35, le rapport molaire oléate d'éthyle/composé tungsténique est de 50 et le rapport molaire composant (b)/composant (a) de 2. Dans l'exemple 33, le rapport molaire composant (c)/composant (a) est égal à 1. La réaction de métathèse de l'oléate d'éthyle est conduite à 85°C. Après 60 minutes de réaction on analyse la phase liquide pour déterminer le rendement en octadécène-9, c'est-à-dire le rapport % du nombre de moles de ce composé formé au nombre de moles d'oléate mis en oeuvre. Le Tableau VIII exprime les résultats obtenus (mêmes abréviations que dans le Tableau I).

## Tableau VIII

| Exemples | Systèmes catalytiques | t (h) | $t_r$ (mn) | Rendement en octadécène-9 (mole %) |
|---|---|---|---|---|
| 34 | $WCl_4$ 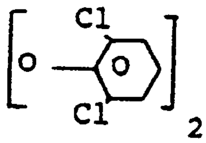 $/SnBu_4$ | 0 | 60 mn | 13 % |
| 35 | idem | 18h | 60 mn | 0 % |
| 33 | $WCl_4$ 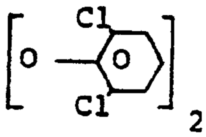 $/SnBu_4/$ $C_8F_{17}CH=CH_2$ | 18h | 60 mn | 13 % |

Les résultats du Tableau VIII montrent qu'un système catalytique de l'art antérieur ne réalise la métathèse de l'oléate d'éthyle que s'il est utilisé immédiatement après sa préparation (exemples 34 et 35). Au contraire, le système catalytique selon l'invention pemet d'effectuer la métathèse de l'oléate d'éthyle même quand il est utilisé 18 heures après sa préparation.

EXEMPLES 36 à 38

Application d'un système catalytique selon l'invention à la métathèse croisée entre des esters d'acide oléfiniques et des oléfines

La réaction étudiée qui est une voie de synthèse de phéromones d'insectes est schématiquement la suivante :

$$\underset{I}{CH_3(CH_2)_7CH = CH(CH_2)_7COOC_2H_5} + \underset{II}{CH_3(CH_2)_3CH = CH(CH_2)_3CH_3}$$

$$\rightleftharpoons$$

$$\underset{III}{C_2H_5OOC(CH_2)_7CH = CH(CH_2)_7COOC_2H_5} + \underset{IV}{CH_3(CH_2)_3CH = CH(CH_2)_7COOC_2H_5}$$

$$+\underset{V}{CH_3(CH_2)_7CH = CH(CH_2)_7CH_3} + \underset{VI}{CH_3(CH_2)_3CH = CH(CH_2)_7CH_3}$$

Cette réaction entre l'oléate d'éthyle et le décène-5 a été réalisée, d'une part en employant un système catalytique selon l'invention

$WCl_4$ 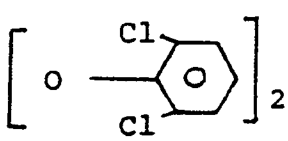 $/SnBu_4/C_8F_{17}CH = CH_2$,

utilisé 200 heures après sa préparation (exemple 36), d'autre part en employant un système catalytique de l'art

antérieur

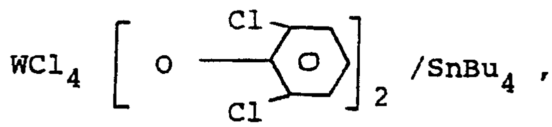

utilisé soit immédiatement (exemple 37), soit 200 heures après sa préparation (exemple 38).

Dans les trois exemples, le rapport molaire décène-5/oléate d'éthyle est égal à 2, et le rapport molaire oléate d'éthyle/composé tungsténique est égal à 50. Les autres conditions sont les mêmes que celles utilisées dans les exemples 33 à 35. Après 90 minutes de réaction à 85°C, on analyse la phase liquide pour déterminer le taux de conversion de l'oléate d'éthyle (I) et le rendement en produit (IV) (calculé en % molaire par rapport à l'oléate de départ). Les résultats sont reportés dans le tableau IX (mêmes abréviations que dans le Tableau I).

TABLEAU IX

| Exemples | Systèmes catalytiques | t (h) | tr (mn) | Conversion de l'oléate (mole %) | Rendement en IV (mole %) |
|---|---|---|---|---|---|
| 36 | $WCl_4$ 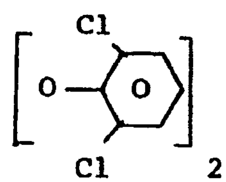 $/SnBu_4$<br>$C_8F_{17}CH = CH_2$ | 200h | 90mn | 85% | 50% |
| 37 | $WCl_4$ 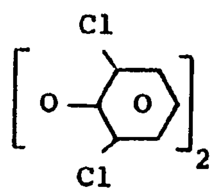 $/SnBu_4$ | 0 | 90mn | 56% | 50% |
| 38 | idem | 200h | 90mn | 0 % | U % |

Ces résultats montrent que contrairement aux systèmes catalytiques de l'art antérieur, le système catalytique selon l'invention permet de réaliser, plus de 8 jours après sa préparation, la réaction de métathèse croisée entre un ester d'acide oléfinique et une oléfine.

## EXEMPLES 39 à 41

### Application d'un système catalytique selon l'invention à la métathèse d'une oléfine cyclique.

Les essais consistent à effectuer la polymérisation par métathèse du dicyclopentadiène, d'une part, avec un système catalytique selon l'invention

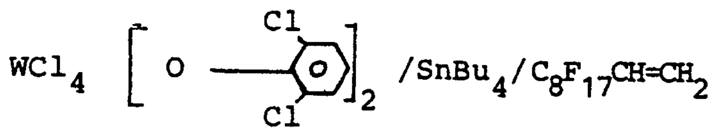

utilisé 200 heures après sa préparation (exemple 39), d'autre part, avec un système catalytique selon l'art antérieur

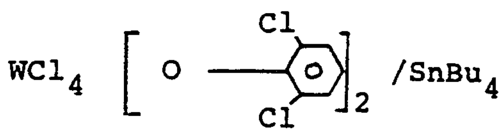

utilisé immédiatement (exemple 40) ou 200 heures après sa préparation (exemple 41).

Dans les trois exemples 39 à 41, les rapports molaires dicyclopentadiène/composé tungsténique sont égaux à 500, et les rapports molaires composant (b)/composant (a) égaux à 2. Dans l'exemple 39, le rapport molaire composant (c)/composant (a) est égal à 1. Les méthodes de préparation et d'utilisation des systèmes catalytiques de l'art antérieur et de l'invention sont les mêmes que celles décrites dans les exemples 1 à 6 précédemment.

La réaction de polymérisation est effectuée à 85°C. Après une heure de réaction, on mesure le rendement en poly-dicyclopentadiène. Les résultats obtenus sont reportés dans le Tableau X (mêmes abréviations que dans le Tableau I).

Les résultats montrent, de façon surprenante, qu'un système catalytique selon l'invention permet de réaliser, plus de 8 jours après sa préparation, la polymérisation par métathèse d'une oléfine cyclique. Dans les mêmes conditions, un système catalytique de l'art antérieur ne montre aucune activité.

TABLEAU X

| Ex. | Systèmes catalytiques | t(h) | $t_r$ (mn) | Rendement en poly-dicyclo-pentadiène (%) |
|---|---|---|---|---|
| 39 | $WCl_4$ 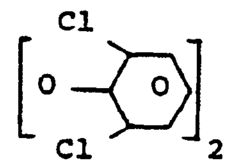 $/SnBu_4$ <br> $C_8F_{17}CH = CH_2$ | 180 h | 60 mn | 100% |
| 40 | $WCl_4$ 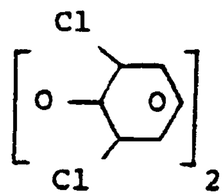 $/SnBu_4$ | 0 | 60 mn | 100% |
| 41 | idem | 180 h | 60 mn | 0% |

L'action des composés fluorés, montrée dans les exemples qui précèdent, est d'autant plus surprenante que les autres halogénures organiques ne permettent pas de stabiliser les catalyseurs au tungstène pour la métathèse. En effet, si des halogénures de Cl, Br ou I, comme décrit dans US 4 262 156, peuvent servir d'initiateurs, en présence d'un composé organo-stannique, ils ne permettent pas de conserver le catalyseur pendant plusieurs heures, avant son utilisation. Selon le brevet US précité un halogénure, efficace en tant qu'initiateur, serait, par exemple, le tétrachlorure de carbone $CCl_4$ (col. 10, Tableau I, essais n° 10 à 13 et 28-30). Il a été essayé dans le cadre de la présente invention, dans l'exemple 42 ci-après, et on a constaté qu'il ne permet pas de conserver le catalyseur pendant quelques heures avec la réaction de métathèse.

EXEMPLE 42

Du pentène-2-cis est soumis à la métathèse exactement comme dans l'exemple 6 donné plus haut et résumé au Tableau I, sauf qu'après les 20 minutes de préparation du catalyseur à 85°C on ajoute $10^{-4}$ mole de $CCl_4$.

Le catalyseur est alors laissé au repos à la température ambiante durant 18 heures.

Utilisé après ce temps, le catalyseur donne seulement un rendement de 0,5% en butène-2, après 90 mn de réaction à 85°C, contre 23% pour l'exemple 1.

## Revendications

1. Système catalytique perfectionné, pour la métathèse d'oléfines, qui comprend au départ (a) un composé halogéné du tungstène, (b) un composé organométallique, alkylique ou arylique, d'un métal autre que W, et (c) un composé organique halogéné,
caractérisé en ce que ce composé (c) est un hydrocarbure aliphatique fluoré ou perfluoré.

2. Système catalytique, selon la revendication 1, caractérisé en ce que le composant (c) est représenté par la formule générale :

$$R_F — R" — R'_F$$

dans laquelle R″ est une-chaîne hydrocarbonée, saturée ou insaturée, possédant de 0 à 7 atomes de carbone; $R_F$ et $R'_F$, identiques ou différents entre eux, sont des chaînes carbonées saturées ou insaturées, partiellement ou totalement fluorées, $R_F$ pouvant posséder de 0 à 20 atomes de carbone, et $R'_F$ de 2 à 20 atomes de carbone.

3. Système catalytique selon la revendication 1 ou 2, caractérisé en ce que le composant (c) répond à la formule $R_F$-R″-$R'_F$ où R" représente une chaîne hydrocarbonée saturée ou insaturée, choisie parmi : $CH_3$-$(CH_2)_n$–, –$(CH2)_{n'}$–, CH2=CH-$(CH2)_m$– et –CH=CH–, n étant un nombre entier de 0 à 6, n′ un nombre entier de 0 à 5 et m un nombre entier de 0 à 3 ; $R_F$ et $R_F'$ sont des chaînes carbonées saturées, totalement fluorées, $R_F$ pouvant posséder de 0 à 12 atomes de carbone et $R'_F$ de 2 à 12 atomes de carbone.

4. Système catalytique selon l'une des revendications 1 à 3, dans lequel le composant(a) est représenté par la formule générale :

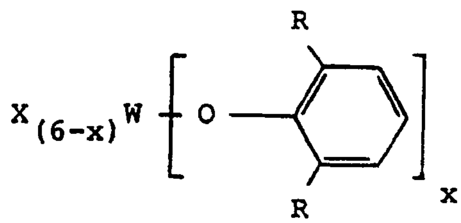

où x = 0 ou 2 ; X est un halogène, de préférence du chlore ou du fluor ; R est un groupe hydrocarboné ou un groupe ou atome électronégatif, de préférence un alkyle en $C_1$ à $C_8$, phényle ou un atome de Cl, Br ou F.

5. Système catalytique selon une des revendications 1 à 4, caractérisé en ce que le composant (b) est un composé organoaluminique, -titanique, -lithien, -magnésien et, de préférence, organo-stannique ou -plombique de formule $M(R'_4)$, M étant un atome de Sn ou Pb et R′ un groupement alkyle ou aryle en $C_1$ à $C_{12}$ et, plus spécialement, un akyle en $C_1$ à $C_5$.

6. Système catalytique selon une des revendications 1 à 5, caractérisé en ce qu'il comprend un solvant organique, notamment benzène, toluène, hydrocarbures saturés en $C_6$ à $C_{22}$, fluorohydrocarbures et, de préférence chlorobenzène.

7. Système catalytique selon une des revendications 1 à 6, caractérisé en ce que le rapport molaire de composant (c)/composant (a) est initialement compris entre 0,5/1 et 20/1.

8. Système catalytique selon une des revendications 1 à 7, caractérisé en ce que le rapport molaire initial de composant (b)/composant (a) est compris entre 1 et 4, et de préférence égal à environ 2.

9. Procédé de préparation d'un système catalytique selon une des revendications 1 à 8, caractérisé en ce qu'il comprend la mise en solution du composant(a) dans un solvant organique approprié, la mise en contact du constituant organométallique (b) avec le composant (a), l'agitation et le chauffage à une température comprise entre 0° et 100°C du mélange réactionnel qui en résulte, pendant 0,5 à 60 minutes, puis introduction d'une quantité efficace du composant (c), et arrêt du chauffage et de l'agitation.

10. Procédé selon la revendication 9, caractérisé en ce que la quantité de composant (c) introduit correspond à un rapport molaire composant (c)/composant (a) compris entre 0,5/1 et 20/1.

11. Procédé selon une des revendications 9 ou 10, caractérisé en ce que le solvant organique est du benzène, toluène, des hydrocarbures saturés en $C_6$ à $C_{22}$, des fréons, ou du chlorobenzène.

12. Procédé selon une des revendications 9 à 11, caractérisé en ce que le temps de chauffage est de 0,5 à 10 minutes lorsque le composant (b) est un organo-plombique, et de 0,5 à 30 minutes dans le cas d'un organo-stannique.

13. Application d'un système catalytique selon une des revendications 1 à 8, à la métathèse d'oléfines portant des groupes fonctionnels ou ne portant pas de tels groupes.

14. Application suivant la revendication 13 à l'obtention de phéromones d'insectes, ou à la polymérisation d'une oléfine cyclique, plus particulièrement, du dicyclopentadiène.

**Ansprüche**

1. Verbessertes katalytisches System für die Metathese von Olefinen, welches anfänglich (a) eine halogenierte Wolframverbindung, (b) eine metallorganische Verbindung, alkylisch oder arylisch, eines anderen Metalls als Wolfram und (c) eine halogenierte organische Verbindung umfasst, dadurch gekennzeichnet, dass diese Verbindung (c) ein aliphatischer fluorierter oder perfluorierter Kohlenwasserstoff ist.

2. Katalytisches System nach Anspruch 1, dadurch gekennzeichnet, dass die Verbindung (c) durch die allgemeine Formel wiedergegeben wird :

$$R_F - R'' - R'F$$

worin R" eine gesättigte oder ungesättige Kohlenwasserstoffkette mit 0 bis 7 Kohlenstoffatomen ist ; RF und R'F, die gleich oder voneinander verschieden sein können, gesättigte oder ungesättigte, teilweise oder völlig fluorierte Kohlenstoffketten sind, wobei RF 0 bis 20 Kohlenstoffatome aufweisen kann und R'F 2 bis 20 Kohlenstoffatome.

3. Katalytisches System nach Anspruch 1 oder 2, dadurch gekennzeichnet, dass die Verbindung (c) der Formel entspricht $R_F$-R"-R'F, worin R" eine gesättigte oder ungesättigte Kohlenwasserstoffkette, ausgewählt aus CH3-$(CH_2)_n$, $-(CH_2)_{n'}-$, $CH_2=CH-(CH_2)_m-$ und $-CH=CH-$ darstellt, n eine ganze Zahl von 0 bis 6 ist, n' eine ganze Zahl von 0 bis 5 und m eine ganze Zahl von 0 bis 3 ; RF und R'F gesättigte, völlig fluorierte Kohlenstoffketten sind, RF 0 bis 12 Kohlenstoffatome aufweisen und R'F 2 bis 12 Kohlenstoffatome.

4. Katalytisches System nach einem der Ansprüche 1 bis 3, worin der Bestandteil (a) durch die allgemeine Formel wiedergegeben wird :

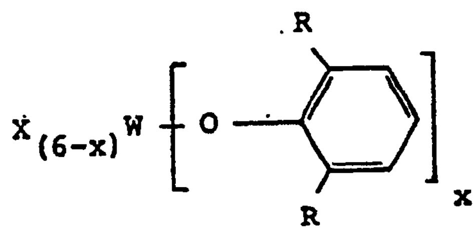

worin x = 0 oder 2 ; X ein Halogen ist, vorzugsweise Chlor oder Fluor ; R eine Kohlenwasserstoffgruppe oder eine elektronegative Gruppe oder ein solches Atom, vorzugsweise $C_1$-bis $C_6$-Alkyl, Phenyl oder ein Cl-, Br- oder F-Atom.

5. Katalytisches System nach einem der Ansprüche 1 bis 4, dadurch gekennzeichnet, dass der Bestandteil (b) eine Organoaluminium-, -titan-, -lithium-, -magnesium- und, vorzugsweise,Organozinn- oder -bleiverbindung der Formel $M(R'_4)$ ist, worin M ein Sn- oder Pb-Atom ist und R' eine Alkyl- oder Arylgruppe von $C_1$ bis $C_{12}$ und, genauer, ein $C_1$- bis $C_5$-Alkyl.

6. Katalytisches System nach einem der Ansprüche 1 bis 5, dadurch gekennzeichnet, dass es ein organisches Lösungsmittel, nämlich Benzol, Toluol, gesättigte Kohlenwasserstoffe von $C_6$ bis $C_{22}$, Fluorkohlenwasserstoffe und, vorzugsweise, Chlorbenzol umfasst.

7. Katalytisches System nach einem der Ansprüche 1 bis 6, dadurch gekennzeichnet, dass das molare Verhältnis Bestandteil (c)/Bestandteil (a) anfänglich zwischen 0,5/1 und 20/1 liegt.

8. Katalytisches System nach einem der Ansprüche 1 bis 7, dadurch gekennzeichnet, dass das anfängliche molare Verhältnis Bestandteil (b)/Bestandteil (a) zwischen 1 und 4 liegt und vorzugsweise gleich oder bei 2 ist.

9. Verfahren zur Herstellung eines katalytischen Systems nach einem der Ansprüche 1 bis 8, dadurch gekennzeichnet, dass es das Inlösungbringen des Bestandteils (a) in einem geeigneten organischen Lösungsmittel, das Inberührungbringen des metallorganischen Bestandteils (b) mit dem Bestandteil (a), das Rühren und Erwärmen auf eine Temperatur zwischen 0° und 100°C der daraus resultierenden Reaktionsmischung über 0,5 bis 60 min, danach die Einführung einei wirksamen Menge des Bestandteils (c) und das Beenden des Erhitzens und des Rührens umfasst.

10. Verfahren nach Anspruch 9, dadurch gekennzeichnet, dass die Menge an eingeführtem Bestandteil (c) einem molaren Verhältnis Bestandteil (c)/Bestandteil (a) zwischen 0,5/1 und 20/1 entspricht.

11. Verfahren nach einem der Ansprüche 9 oder 10, dadurch gekennzeichnet, dass das organische Lösungsmittel aus Benzol, Toluol, gesättigten Kohlenwasserstoffen von $C_6$ bis $C_{22}$, Freonen oder Chlorbenzol ist.

12. Verfahren nach einem der Ansprüche 9 bis 11, dadurch gekennzeichnet, dass die Heizzeit 0,5 bis 10 min beträgt, wenn der Bestandteil (b) eine Organobleiverbindung ist, und 0,5 bis 30 min im Fall einer Organozinnverbindung.

13. Verwendung eines katalytischen Systems nach einem der Ansprüche 1 bis 8 für die Metathese von Olefinen, die funktionelle Gruppen aufweisen oder solche Gruppen nicht aufweisen.

14. Verwendung nach Anspruch 13 zur Herstellung von Insektenpheromonen oder für die Polymerisation eines cyclischen Olefins, insbesondere von Dicyclopentadien.

## Claims

1. Improved catalytic system for the metathesis of olefins, comprising initially (a) a halogen compound of tungsten, (b) an organo-metallic compound of a metal other than W and (c) a halogenated organic compound, characterised in that (c) is a fluorinated or perfluorinated organic product.

2. Catalytic system according to claim 1, characterised in that the component (c) is represented by the general formula :

$$R_F - R'' - RF'$$

in which R" is a saturated or unsaturated hydrocarbon chain having 0 to 7 carbon atoms, $R_F$ and RF' are the same or different and are saturated or unsaturated, partially or totally fluorinated carbon chains, $R_F$ if required having 0 to 20 carbon atoms and RF 2 to 20 carbon atoms.

3. Catalytic system according to claim 1 or 2, characterised in that the component (c) corresponds to the formula

$$RF\text{-}R''\text{-}RF'$$

where R" represents a saturated or unsaturated hydrocarbon chain selected from

$$CH_3\text{-}(CH_2)n\text{–},\ \text{–}(CH_2)n'\text{–},\ CH_2{=}CH\text{-}(CH_2)m\text{–} \text{ and } \text{–}CH{=}CH\text{–},$$

n being an integral number from 0 to 6, n' being an integral number from 0 to 5 and m an integral number from 0 to 3, $R_F$ and RF totally fluorinated saturated carbon chains, RF possibly possessing 0 to 12 carbon atoms and $R'_F$ 2 to 12 carbon atoms.

4. Catalytic system according to any of claims 1 to 3, in which the component (a) is represented by the general formula :

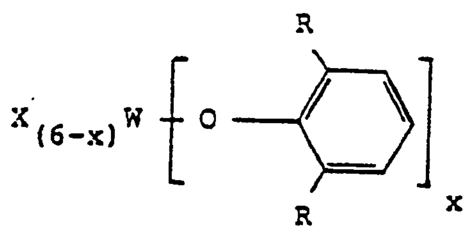

where x = 0 or 2, X is a halogen, preferably chlorine or fluorine, R is a hydrocarbon group or an electro-negative group or atom, preferably a $C_1$ to $C_8$ alkyl, phenyl or an atom of Cl, Br or F.

5. Catalytic system according to any of claims 1 to 4, characterised in that the component (b) is an organo-aluminium, titanium, lithium or magnesium compound, preferably organo-tin or lead, of the formula $M(R'_4)$, M being an atom of Sn or Pb and R' a $C_1$ to $C^{12}$ alkyl or aryl group, more particularly $C_1$ to $C_5$ alkyl.

6. Catalytic system according to any of claims 1 to 5, characterised in that it comprises an organic solvent, notably benzene, toluene, $C_6$ to $C_{22}$ saturated hydrocarbons, fluoro-hydrocarbons and, preferably, chlorobenzene.

7. Catalytic system according to any of claims 1 to 6, characterised in that the molar ratio of component (c)/component (a) is initially between 0.5/1 and 20/1.

8. Catalytic system according to any of claims 1 to 7, characterised in that the initial molar ratio of component (b)/component (a) is from 1 to 4 and preferably is equal to about 2.

9. Process of preparation of a catalytic system according to any of claims 1 to 8, characterised in that it comprises putting into solution component (a) in an appropriate organic solvent, contacting the organo-metallic

constituent (b) with component (a), agitation and heating of the reaction medium which results to a temperature in the range from 0° to 100°C for 0.5 to 60 minutes, then introduction of an effective quantity of component (c) and cessation of the heating and agitation.

10. Process according to claim 9, characterised in that the quantity of the component (c) introduced corresponds to a molar ratio of component (c)/component (a) between 0.5/1 and 20/1.

11. Process according to either of claims 9 or 10, characterised in that the organic solvent is benzene, toluene, $C_6$ to $C_{22}$ saturated hydrocarbons, freons or chlorobenzene.

12. Process according to any of claims 9 to 11, characterised in that the heating time is from 0.5 to 10 minutes when the component (b) is ortho-plumbic and 0.5 to 30 minutes in the case of a organo-tin compound.

13. Use of a catalytic system according to any of claims 1 to 8 for the metathesis of olefins containing functional groups or not carrying such groups.

14. Use according to claim 13 for the production of insect pheromones or for the polymerisation of a cyclic olefin, more particularly dicyclopentadiene.